# EUROPÄISCHE PATENTANMELDUNG

(11) **EP 1 707 351 A1**
(43) Veröffentlichungstag der Anmeldung: **04.10.2006**
(21) Anmeldenummer: 06004098.7
(22) Anmeldetag: 01.03.2006
(51) Int. Cl.: B32B 37/14, B32B 38/18

(54) **Verfahren und Vorrichtung zur Herstellung von querelastischen mehrschichtigen Materialbahnen**

(30) Priorität: 10.03.2005 DE 102005011059
(71) Anmelder: RKW AG Rheinische Kunststoffwerke, 67547 Worms (DE)
(72) Erfinder: Epping, Reinhard, 48599 Gronau (DE)
(74) Vertreter: Zellentin, Wiger

(57) **Zusammenfassung**

Die vorliegende Erfindung betrifft ein Verfahren zur Herstellung von querelastischen Materialbahnen, bestehend aus einer Oberlage, einer Unterlage sowie einer vorgereckten, mit dem Ober- Und Untervliesstoff (9,10) verbundenen elastischen Zwischenlage (7), bei welchem man den Obervliesstoff und den Untervliesstoff über Walzen zwischen zwei seitlichen, mit Haken (5) versehenen umlaufenden Bändchentransportketten (1,2) zusammenbringt, mit Hilfe eines Bändchenlegers (6) elastische Bändchen (7) zwischen den Transportketten (1,2) hin- und herführt und dabei um die Haken (5) schlingt, so dass parallele Lagen quer zur Umlaufrichtung der Transportketten entstehen, wobei die Bändchen vorgereckt sind. Oberlage und Unterlage werden gegen die Bändchen gedrückt und die Lagen miteinander verklebt oder verschweißt.

## Beschreibung

Die vorliegende Erfindung betrifft die Herstellung von dehnbaren elastischen mehrlagigen Materialbahnen für z.B. Kleidungsstücke, insbesondere aber für Hygieneprodukte.

Aus der eigenen älteren Patentanmeldung DE 103 51 831 ist ein Verfahren zur Herstellung mehrschichtiger dehnbarer Materialbahnen bekannt, die bei der Herstellung eine Elastizität quer zur Richtung ihrer Herstellung aufweisen, so dass sie in Produktionsrichtung abgefördert und weiter verarbeitet, d.h. mit Zuschnitten für Hygieneprodukte verbunden werden können.

Diese Lösung und der dort aufgeführte Stand der Technik sind auf die Verwendung elastischer Folien beschränkt. Die Dehnbarkeit von Folien ist jedoch begrenzt, es hat sich daher die Aufgabe gestellt, eine Möglichkeit zur Erhöhung der Dehnbarkeit derartiger Flächengebilde zu finden, wobei die Möglichkeit der direkten Weiterverarbeitung in Förderrichtung der Produktionsanlage aufrecht erhalten bleiben soll.

Die Lösung dieser Aufgabe gelingt mit dem Verfahren zur Herstellung von elastischen Flächengebilden, bestehend aus einer Oberlage, einer Unterlage sowie einer vorgereckten, mit der Ober- und Unterlage verbundenen elastischen Zwischenlage, bei welchem man die Oberlage und die Unterlage über Walzen zwischen zwei seitlichen, mit Haken versehenen umlaufenden Bändchentransportketten zusammenbringt, mit Hilfe eines Bändchenlegers elastische Bändchen zwischen den Transportketten hin- und herführt und dabei um die Haken schlingt, so dass parallele Lagen quer zur Umlaufrichtung der Transportketten entstehen, wobei die Bändchen vorgereckt sind. Oberlage und Unterlage werden gegen die Bändchenlage gedrückt und die Lagen miteinander verklebt oder verschweißt.

Auf diese Weise gelingt es, einen Verbund aus z.B. einem Obervliesstoff und einem Untervliesstoff herzustellen, welcher elastisch ist. Verwendet man dabei Lagen, die selbst dehnbar sind, so kann das nach der Herstellung durch Kontraktion der elastischen Bändchen geraffte Material auch über die ungeraffte Materialgröße der Lagen hinaus gedehnt werden.

Die Art des Lagenmaterials kann dabei in weiten Grenzen variiert werden, sie können gleich oder unterschiedlich sein und z.B. neben den für Hygiene- und medizinische Zwecke bevorzugten Vliesstoffen thermoplastische Folien, Papier oder textiles Material sein, ebenso sind Kombinationen davon möglich.

Die nachfolgenden Ausführungen beziehen sich auf die Verwendung von Vliesstoff als Ober- und Unterlage des Verbundmaterials.

Da die-elastischen Bändchen wesentlich besser gestreckt werden können und damit einen weiteren Bereich der Elastizität aufweisen, wird hierdurch auch der Anpassungs- und Tragekomfort von Hygieneprodukten, wie z.B. Windelhosen erhöht und über einen größeren Bereich ein dichter Abschluss z.B. bei Verwendung als Windelbündchen/Gürtel um den Bauch des Verbrauchers oder auch ggf. um die Oberschenkel erzielt.

Das Vorstrecken der gummielastischen Bändchen erfolgt dabei z.B. so, dass man die Bändchen über eine hin- und herbewegte Rolle umlenkt und diese mit einer Streckeinheit dehnt.

Dabei können grundsätzlich einzelne Endlosbändchen um die Haken gelegt werden, vorteilhaft ist das Ablegen mehrerer parallel laufender Bändchen gleichzeitig, wobei der Ablegeschritt dem Gesamtabstand der entsprechenden Hakenzahl auf den Transportketten entspricht.

Das Strecken der Bändchen kann dabei mit Hilfe eines Rollenpaares erfolgen, zwischen denen das oder die Bändchen im Reibschluss laufen und dadurch einen Widerstand zum Bändchenleger erzeugen, wobei sich der Bändchenleger quer zu den Bändchentransportketten bewegt und das Rollenpaar synchron zu diesem.

Bei diesem Verfahren entstehen seitlich neben den Lagen Schlingen.

Die Lagen werden nach dem Zusammenbringen einer Schweiß- oder Klebestation zugeführt, die z.B. eine Nipeinheit aufweist, die die beiden Vliesstofflagen, welche vorher mit einem Kleber auf der Innenseite versehen wurden und die Bändchen verpresst werden, so dass diese mit den beiden Vliesstofflagen verkleben. Vorgezogen wird jedoch ein Verschweißen unter Ultraschalleinwirkung, wobei oberhalb der Andruckwalze eine Sonotrode angeordnet und die Andruckwalze entsprechend profiliert ist.

Das Klebemittel kann ein Hotmeltkleber, insbesondere ein elastischer Hotmeltkleber sein, als Vliesstoffmaterial kommt in beiden Fällen z.B. ein Nonwoven aus einem Polyolefin zur Anwendung.

Der verklebte oder verschweißte Verbund wird letztlich einer Schneideinheit zugeführt, die die seitlich überstehenden Schlingen abtrennt und ggf. die Materialbahn in die gewünschte Breite teilt. Daher ist die Bahnbreite der Vliese entsprechend geringer als der Abstand der Bändchentransportketten voneinander, um Platz für den Eingriff des Schneidwerkzeugs zu lassen.

Ein weiterer besonderer Vorteil der erfindungsgemäßen Vorgehensweise liegt darin, dass auf diese Weise ein sehr breiter Schichtverbund herstellbar ist und die Flächengebilde eine entsprechende Länge aufweisen, so dass damit ohne weiteres Hygieneprodukte, z.B. Bündchen, Seitenteile oder Gürtel für Inkontinenzwindeln herstellbar sind, was mit den Mitteln des Standes der Technik nicht erreichbar war.

Die Bändchen bestehen dabei vorzugsweise aus z.B. Isopren (Fulflex) oder anderen Elastomeren die als Flachbändchen, Multifilamenten oder als Direktextrudat der Vorrichtung zugeführt werden.

Auch bei den elastischen Multifilamenten (Lycra) kann die Ultraschalltechnik verwendet werden. Die Filamente werden dabei unter der Sonotrode soweit auseinander Treiben, dass eine Verbindung zwischen den beiden Lagen, insbesondere Vlieslagen erzeugt werden kann.

Direktextrudate (z.B. Monofilamente) werden alternativ mittels Hotmelt - Kleber zwischen den beiden Lagen verklebt.

Die erfindungsgemäße Vorrichtung besteht damit aus zwei seitlich umlaufenden Transportketten, die an den Obertrums nach oben abstehende Haken aufweisen, die z.B. pilzförmig ausgebildet sind, wobei die Köpfe das Abrutschen der Bändchen nach oben verhindern. Weiterhin weist die Vorrichtung Umlenkrollen für die Zustellung von Ober- und Untervliesstoff auf sowie einen hin- und herbeweglichen Bändchenleger. Dieser Bändchenleger kann in Bezug auf die Transportrichtung der Bändchentransportketten ortsfest sein, wobei dann letztere schrittweise bewegt werden, oder aber der Bändchenleger ist zusätzlich vor- und zurücklaufend ausgebildet, so dass der Transportvorgang nicht unterbrochen werden muss.

Zusätzlich besitzt die Vorrichtung eine Streckeinheit, welche beim Ablegen der Bändchen die gewünschte Vorspannung schafft sowie eine Schweiß- oder Klebestation, an die eine Schneideinheit beidseitig des hergestellten Schichtmaterials und zwischen dieser und den Transportketten anschließt.

Eine solche Vorrichtung wird anhand der beiliegenden Figuren näher erläutert.

Dabei zeigen
Fig. 1 die Vorrichtung in Seitenansicht und
Fig. 2 diese in dreidimensionaler Darstellung als Ausschnitt.

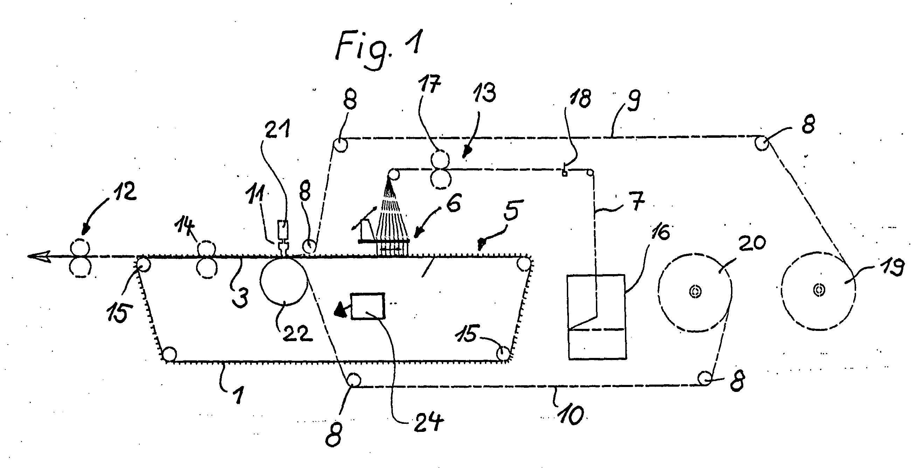

In Fig. 1 ist eine der beiden Bändchentransportketten 1; 2 gezeigt, die über Umlenkrollen 15 im Kreis geführt und motorisch angetrieben ist. Diese Transportketten 1; 2 weisen Stacheln mit Köpfen oder Umbiegungen auf, die Häkchen 5 bilden.

Aus einem Vorratsbehälter 16 wird den Häkchen 5 eine Vielzahl paralleler Bändchen 7 zugeführt. Diese gelangen zunächst zu einem Kamm 18, in welchem sie auf gewünschten Abstand zueinander gebracht werden und von dort zu einem Rollenpaar 17, zwischen denen die Bändchen 7 im Reibschluss laufen. Dieses Rollenpaar 17 dient der Vorstreckung der Bändchen 7, unter welcher diese' zwischen Haken 5 der Bändchentransportketten (1, 2) hin- und herbewegt werden.

Dazu werden die Bändchen 7 einem Bändchenleger 6 zugeführt, welcher quer zur Transportrichtung der Transportkette 1 verfahrbar ist und nach Erreichen der Haken 5 eine relative Rückwärtsbewegung erfährt, um versetzt um die Breite des Hakenschaftes und der Bändchenlage in umgekehrte Richtung zu den gegenüberliegenden Haken bewegt zu werden, wonach der Bändchenleger 6 wieder in die Gegenrichtung läuft.

Das Strecken der Bändchen 7 kann dabei so geschehen, dass man diese nach dem Rollenpaar 17 über eine Umlenkung führt, die ortsveränderlich ist, um den Weg der Bändchen 7 entsprechend der Bewegungsrichtung des Bändchenlegers 6 zu verlängern oder zu verkürzen. Alternativ kann das Rollenpaar 17 selbst (auf und ab) bewegt werden.

Die Bändchenlage wird von den Bändchentransportketten 1, 2 einer Schweiß-/Klebestation 11 zugestellt. Zuvor werden über Umlenkrollen 8 von Vorratswalzen 19; 20 den Obervliesstoff 9 und den Untervliesstoff 10 mit der Bändchenlage vereinigt und in der Schweiß-/Klebestation 11 vereinigt. Diese besteht z.B. aus einer oberen Sonotrode 21 und einer unteren profilierten Andruckwalze 22, in welcher die Lagen unter Ultraschalleinwirkung miteinander verschweißt oder verklebt werden. Bei Verwendung von elastischen vielfaserigen Filamenten werden diese dabei flachgedrückt und flächig mit den Vlieslagen verschweißt.

Bei Verwendung von Hotmeltklebern wird das Material an den erforderlichen Stellen mit heißem Kleber benetzt und gegeneinander gedrückt, bis der Kleber genug ausgehärtet ist. Dies kann z.B. mit Hilfe eines Walzenpaares geschehen.

Nach dem Verkleben oder Verschweißen gelangt das entstandene Schichtmaterial (vorgespannt) in den Eingriff einer Schneideinheit, die die seitlichen-überstehenden Bändchenschlingen abtrennt. Über das Abzugswerk 12 letztlich wird das fertige Produkt abgezogen.

Bei Verwendung von elastischen Multifilamenten werden diese dabei so weit auseinander getrieben, das eine Verschweißung der beiden Vliesstofflagen erfolgen kann.

Bei der Verwendung von Homeltklebern wird das Material an den erforderlichen Stellen mit heißem Kleber benetzt und gegeneinander gedrückt, bis der Kleber genug ausgehärtet ist.

Bei der Verwendung von Klebstoffen zur gleichzeitigen Verbindung von Obermaterial 9 und Untermaterial 10 kann (dauerelastischer) Hotmeltkleber mit einer Breitschlitzdüse 24 - oder alternativ über eine hin- und herwandernde Einfachdüse (nicht gezeigt) - vor der Walze 22 auf das Untermaterial 10 (Vlies) aufgetragen werden, wobei dann anstelle der Sonotrode 21 eine Kühlung, z.B. mit Hilfe einer Walze vorzusehen ist, die den Hotmeltkleber rasch aushärten lässt, so dass die gummielastischen Bändchen sofort fest mit dem Obermaterial 9 und dem Untermaterial 7 verklebt werden. Die Bändchen können dabei bis zu etwa 300 % vorgereckt sein.

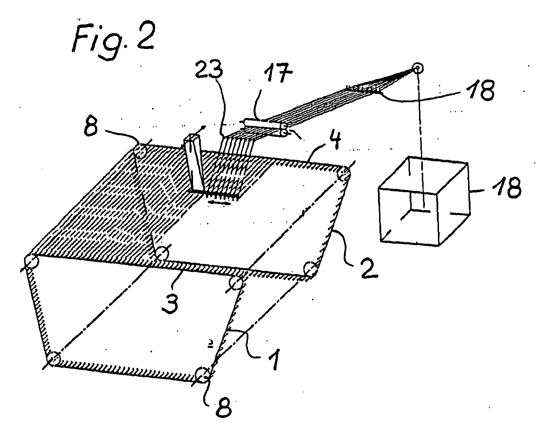

In Fig. 2 ist der Vorgang des Bändchenlegens als Detail der Anlage dreidimensional dargestellt. Man erkennt die beiden Transportketten 1; 2, denen die aus dem Vorratsbehälter 16 abgeförderten parallelen Bändchen 7 über einen Kamm 18 und dem Rollenpaar 17 der Streckeinheit 13 über eine Brücke 23 und den Bändchenleger 6 zugestellt wird.

Sowohl diese Brücke 23 als auch das Rollenpaar 17 können zur Wegverlängerung und Wegverkürzung bei der Vorspannung dienen, wozu sie mit vertikalem Vektor verfahrbar sind. Außerdem kann das Rollenpaar 17, das gleichzeitig dem Abfördern aus dem Vorratsbehälter 16 dienen kann, mit variabler Umlaufgeschwindigkeit gesteuert sein.

Nach dem Verkleben oder Verschweißen gelangt das entstandene Schichtmaterial (vorgespannt) in den Eingriff einer Schneideinheit, die die seitlichen überstehenden Bändchenschlingen abtrennt, und die Materialbahn in die gewünschte Breite schneidet.

### Bezugszeichenliste

- 1, 2: Transportketten
- 3, 4: Obertrums
- 5: Haken
- 6: Bändchenleger
- 7: Bändchen
- 8: Umlenkrollen
- 9: Obervliesstoff
- 10: Untervliesstoff
- 11: Schweiß-/Klebestation
- 12: Abzugswerk
- 13: Streckeinheit
- 14: Schneideinheit
- 15: Umlenkrollen
- 16: Vorratsbehälter
- 17: Rollenpaar
- 18: Kamm
- 19, 20: Vorratswalzen
- 21: Sonotrode
- 22: Andruckwalze
- 23: Brücke
- 24: Breitschlitzdüse

## Patentansprüche

1. Verfahren zur Herstellung von querelastischen Materialbahnen, bestehend aus einer Oberlage, einer Unterlage sowie einer vorgereckten, mit den Vlieslagen verbundenen elastischen Zwischenlage, **dadurch gekennzeichnet, dass** man die Oberlage und die Unterlage über Walzen zwischen zwei seitlichen, mit Haken versehenen umlaufenden Bändchentransportketten zusammenführt, mit Hilfe eines Bändchenlegers elastische Bändchen zwischen den Transportketten hin- und herführt und dabei um die Haken schlingt, so dass parallele Lagen quer zur Umlaufrichtung der Transportketten entstehen, wobei die Bändchen vorgereckt sind, Oberlage und Unterlage gegen die Bändchenlage drückt und die Lagen miteinander verklebt oder verschweißt.

2. Verfahren nach Anspruch 1, **dadurch gekennzeichnet, dass** man die Bändchen über eine obere ebenfalls hin- und herbewegte Rolle umlenkt und die Bändchen mit Hilfe einer Streckeinheit definiert vorstreckt.

3. Verfahren nach Anspruch 2, **dadurch gekennzeichnet, dass** eine Streckeinheit verwendet wird, die ein hin- und herbewegliches und/oder mit variabler Geschwindigkeit antreibbares Rollenpaar ist, zwischen denen die Bändchen im Reibschluss laufen.

4. Verfahren nach Anspruch 1 - 3, **dadurch gekennzeichnet, dass** die seitlichen, die entstehenden Bändchenschlingen aufweisenden Ränder in einer Schneideinheit abgetrennt werden, wonach man das Produkt über ein Abzugswerk abzieht.

5. Verfahren nach Anspruch 1 - 4, **dadurch gekennzeichnet, dass** man die Bändchen über einen Bändchenleger den Transportketten zuführt, welcher in Produktionsrichtung vor und zurück sowie seitlich hin- und herbewegt wird.

6. Verfahren nach Anspruch 1 - 5, **dadurch gekennzeichnet, dass** als Oberlage (9) und die Unterlage (10) ein Vliesstoff verwendet wird.

7. Verfahren nach Anspruch 1-6, **dadurch gekennzeichnet, dass** als Bändchen ein Flächenbändchen aus einem Elastomer wie Isopren oder aus einem elastischen Multifilament verwendet wird, wobei man ein solches Filament mit Hilfe von Profilierungen der Andruckwalze gegen eine Sonotrode drückt, hierbei den Faserstrang flächig aufweitet und diese aufgeweitete Fläche mit Hilfe von Ultraschall mit dem Ober- und Untermaterial verschweißt.

8. Verfahren nach Anspruch 1 - 7, **dadurch gekennzeichnet, dass** man ein Direktextrudat-Filament einsetzt und dieses mit einem Hotmeltkleber zwischen den Lagen verklebt.

9. Vorrichtung zur Herstellung von elastischen Bündchen, bestehend aus einem Obervliesstoff sowie einer vorgereckten, mit dem Ober - und Untervliesstoff verbundenen elastischen Zwischenlage, **gekennzeichnet durch** die folgenden Merkmale:
a. es sind zwei umlaufende Bändchentransportketten vorgesehen, die an deren Obertrums nach oben abstehende Haken aufweisen,
b. oberhalb der Bändchentransportketten befindet sich ein quer zur Laufrichtung der Bändchentransportketten hin- und herbewegbarer Bändchenleger zur Ablage elastischer vorgereckter Bändchen zwischen die gegenüberliegenden Haken,
c. über Umlenkrollen sind Obervliesstoff und Untervliesstoff oberhalb und unterhalb der Bändchenlage zueinander führbar und laufen
d. zu einer Schweiß-/Klebestation zusammen, in der
e. die Lagen und Bändchen miteinander verbindbar sind, an das sich
f. ein Abzugswerk anschließt.

10. Vorrichtung nach Anspruch 9, **dadurch gekennzeichnet, dass** der Bändchenleger längs und quer zur Transportrichtung des Bändchentransportkettenpaares verfahrbar ist.

11. Vorrichtung nach Anspruch 9 oder 10, **gekennzeichnet durch** eine Schneideinheit zum Abschneiden der über die Lagen überstehenden Schlingen des elastischen Bändchenmaterials.
